# FASCICULE DE BREVET EUROPEEN

(11) **EP 0 625 917 B1**
(45) Date de publication et mention de la délivrance du brevet: **25.03.1998**
(21) Numéro de dépôt: 94900706.6
(22) Date de dépôt: 15.12.1993
(51) Int. Cl.: A61M 5/24, A61J 1/00, A61M 5/50

(54) **DISPOSITIF POUR LA PREPARATION D'UNE SOLUTION, D'UNE SUSPENSION OU D'UNE EMULSION D'UNE SUBSTANCE MEDICINALE**
VORRICHTUNG ZUR VORBEREITUNG EINER LÖSUNG, EINER SUSPENSION ODER EINER EMULSION EINER MEDIZINISCHEN LÖSUNG
DEVICE FOR PREPARING A MEDICINAL SUBSTANCE SOLUTION, SUSPENSION OR EMULSION

(30) Priorité: 15.12.1992 FR 9215072; 23.12.1992 FR 9215699
(43) Date de publication de la demande: 30.11.1994
(73) Titulaire: SANOFI, 75008 Paris (FR)
(72) Inventeur: Meyer, Gabriel, CH-1195 Dully (CH)
(74) Mandataire: Nithardt, Roland
(86) Numéro de dépôt international: CH9300282
(87) Numéro de publication internationale: WO9413344

(56) Documents cités:
- FR-A- 2 625 981
- GB-A- 1 120 897
- US-A- 3 841 329
- US-A- 4 014 330
- US-A- 4 180 070
- US-A- 4 909 795

## Description

La présente invention concerne un dispositif pour la préparation d'une solution, d'une suspension ou d'une émulsion d'une substance médicinale active, à partir d'au moins deux composants dont l'un au moins est à l'état liquide.

La reconstitution avant usage d'un médicament à partir d'un ou de plusieurs composants stockés dans un même ensemble hermétiquement fermé et destinés à être mélangés dans cet ensemble, sans introduction d'éléments extérieurs pose de multiples problèmes. Un grand nombre de flacons à deux composants a été développé à cet effet. Ces flacons comportent généralement un conteneur unique séparé en deux compartiments par un organe d'obturation mobile. Les deux compartiments sont hermétiquement fermés pendant le stockage. Avant l'utilisation du médicament reconstitué, une étape d'activation du système permet de mélanger les substances dont au moins une est à l'état liquide. Dans ce but l'organe d'obturation mobile qui sépare les deux compartiments est poussé de façon à pénétrer dans une zone élargie, ou pourvue d'un by-pass, permettant l'écoulement du liquide ou solvant vers l'autre composant qui peut être liquide, solide, pâteux, etc..

La fabrication de ces conteneurs doubles n'est pas toujours aisée et ne répond pas toujours aux besoins de l'industrie pharmaceutique pour une application particulière. Une seringue à deux composants du type susmentionné a fait l'objet du brevet US N^{º} 4 014 330 qui décrit une seringue à deux composants contenus respectivement dans deux compartiments obturés par deux obturateurs ayant également une fonction de piston. Ce dispositif est construit en deux éléments qui doivent être assemblés avant usage. La mise en place du flacon qui définit le compartiment distal, correspond obligatoirement avec l'activation du système. La reconstitution et le transfert ne peuvent pas se faire en milieu fermé, d'où des risques importants de contamination du personnel soignant par le produit, et de perte de stérilité du mélange. De plus le produit actif est contenu dans le conteneur proximal ouvert à ses deux extrémités et muni d'ailettes. Cela pose notamment des problèmes de production lors du conditionnement d'un lyophilisat, d'une poudre ou d'un liquide.

En effet il est impératif de conditionner ces produits actifs dans un flacon cylindrique à fond plat scellé à la flamme pour respecter les exigences particulières de conditionnement de ces substances avec des équipements connus en soi, lors de la préparation à vide (lavage, siliconage, fixation du film de silicone, dépyrogénisation et stérilisation dudit flacon), son remplissage (liquide, poudre, lyophilisat), sa fermeture (bouchonnage) et son transfert en direction d'une unité d' assemblage avec le composant comportant le compartiment contenant le diluant.

Le système tel que décrit dans le brevet US 4 014 330 ne peut pas satisfaire à ces exigences, car le produit actif devrait être conditionné dans le conteneur distal et, dans ces conditions, en raison de sa construction, la reconstitution n'est plus possible.

Un des problèmes qui se pose tout particulièrement, et qui ne peut que difficilement être résolu par les systèmes où les composants sont contenus dans un conteneur unique à deux compartiments, est celui du conditionnement individuel des deux composants. En d'autres termes, les conditions de préparation et de stérilisation de l'un des composants ne sont pas forcément celles qui conviennent à la préparation de l'autre. Un solvant ne peut pas être traité de la même manière qu'un lyophilisat ou que certaines poudres. Le lyophilisat ne résiste pas à la chaleur nécessaire pour stériliser un solvant par autoclavage. Dans un conteneur unique, le lyophilisat est séparé du solvant par un obturateur mobile en élastomère. Le solvant est en contact permanent avec cet obturateur mobile, si bien que la pression partielle de vapeur est constamment à son maximum. Le risque de pénétration de vapeur à travers cet obturateur mobile est grand et la durée de conservation du lyophilisat peut être compromise ou limitée dans le temps. En conséquence le choix des élastomères est limité à celui dont la capacité de barrière étanche est la plus élevée. Cette dernière contrainte impose l'établissement en temps réel de preuves de stabilité et de compatibilité ce qui est fort coûteux et long et handicape les entreprises pharmaceutiques lorsqu'elles doivent prendre une décision relative au conditionnement de leurs produits dans des systèmes à deux chambres.

A ces inconvénients, s'ajoute le risque accru de perte de la stérilité du conteneur à cause du milieu humide. Dans des conditions de conditionnement monocompact, un produit sec, par ailleurs non autoclavable, a moins de chances de laisser croître les germes s'il se trouve dans un milieu sec. Cette absence de risques disparaît dès le moment où, dans un même conteneur, on conditionne un liquide. L'addition d'agents conservateurs n'est pas souhaitée, et même interdit dans certains cas, notamment pour les produits à usage unique.

Les produits à usage multiple, en doses successives, sont conditionnés avec des agents conservateurs, non pas pour limiter la croissance des germes pendant le stockage, mais pour maintenir l'état stérile après reconstitution pendant toute la période d'utilisation qui peut durer plusieurs jours.

Un autre problème qui se pose et qui ne peut que difficilement être résolu par les systèmes existants est la maîtrise du débit lors de la reconstitution du mélange sans action externe par un procédé de vissage tel que décrit dans le brevet européen N^{º} 0 298 067 B1.

La présente invention se propose de pallier ces inconvénients en réalisant un dispositif simple et efficace dans lequel les risques de contamination d'un des composants par l'autre sont supprimés, dans lequel les composants de base nécessaires à la préparation d'un mélange de traitement peuvent être soumis à des opérations distinctes en vue de leur préparation et de leur conditionnement, et dans lequel le débit de reconstitution est maîtrisé en milieu étanche et stérile sans action externe.

Ce but est atteint par le dispositif de l'invention tel que défini par la revendication 1.

Dans une forme de réalisation avantageuse, le dispositif peut comporter un manchon dans lequel est logé le conteneur proximal et au moins partiellement le conteneur distal.

Ce manchon peut être associé à un capuchon poussoir et à un mécanisme de dosage pour l'application de doses multiples.

Selon une autre forme de réalisation, ledit manchon peut être réalisé d'une pièce par moulage avec le conteneur proximal.

Suivant l'application à laquelle est destiné le dispositif, ledit manchon est pourvu d'un embout constituant un applicateur de gouttes ophtalmique ou un pulvérisateur nasal, intégré au conteneur proximal.

Dans la forme de réalisation préférée, ledit manchon se compose d'un premier élément tubulaire et d'un second élément tubulaire emboîtables l'un dans l'autre, le premier élément tubulaire contenant ledit conteneur proximal et ledit second élément secondaire contenant ledit conteneur distal.

De préférence, ledit premier élément tubulaire comporte des crans et ledit second élément tubulaire comporte au moins deux languettes agencées pour coopérer avec lesdits crans.

Selon l'invention, la tige de transfert comporte des moyens d'accouplement étanches et stériles avec lesdits obturateurs-pistons-vannes, ces moyens étant constitués par lesdits embouts solidaires de la tige de transfert.

Lorsque le dispositif est en position de stockage, lesdits obturateurs-pistons-vannes sont agencés pour assurer une fermeture étanche et stérile des conteneurs correspondants, par appui contre les parois intérieures de ces conteneurs de telle manière que ces conteneurs peuvent être stockés et traités indépendamment.

Dans toutes les formes de réalisation ladite tige de transfert est agencée pour assurer un accouplement rigide étanche et stérile desdits obturateurs-pistons-vannes rendus solidaires desdits conteneurs distal et proximal.

Dans le dispositif selon l'invention, ledit conteneur proximal est agencé pour pouvoir recevoir divers embouts et applicateurs en fonction des utilisations thérapeutiques prévues.

La présente invention sera mieux comprise en référence à la description d'exemples de réalisation préférés et aux dessins annexés dans lesquels :
les figures 1 à 4 représentent une forme de réalisation avantageuse du dispositif selon l'invention successivement pendant les phases de stockage, d'activation et d' utilisation,
la figure 5 illustre une forme de réalisation particulière du dispositif destinée à une utilisation comme distributeur de gouttes ophtalmiques,
les figures 6 à 9 représentent une autre forme de réalisation du dispositif de l'invention successivement au stockage à l'activation au début et à la fin de l'utilisation,
les figures 10 à 13 représentent une variante constituant un perfectionnement du dispositif tel que représenté par les figures 1 à 4,
les figure 14 à 18 illustrent une autre forme de réalisation du dispositif utilisé comme seringue associé à un mécanisme de dosage pour l'application de doses multiples,
les figures 19 à 22 illustrent le dispositif selon l'invention adapté au domaine ophtalmique,
les figures 23 et 24 représentent des vues agrandies des obturateurs-pistons-vannes en position de repos,
les figures 25 et 26 représentent des vues agrandies des obturateurs-pistons-vannes dans la position d'activation et d'utilisation,
les figures 27 à 30 représentent des vues agrandies d'accessoires adaptables au dispositif en fonction des utilisations prévues et
les figures 31 à 62 représentent schématiquement les différentes phases de préparation et d'assemblage des composants constitutifs du dispositif selon l'invention dans les différentes utilisations de ce dispositif et avec les différents composants de base correspondant à ces utilisations.

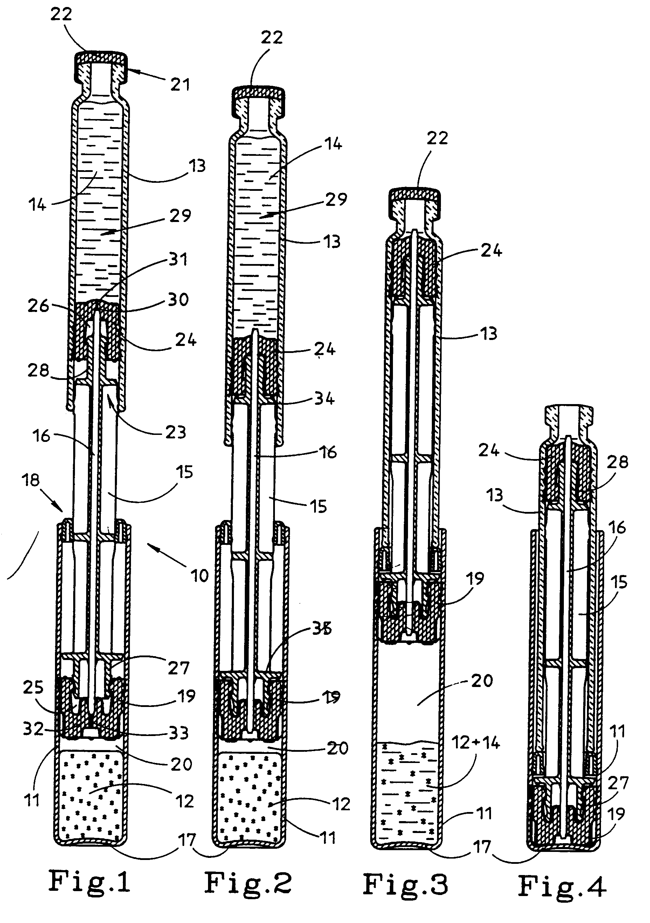

Les figures 1 à 4 représentent une forme de réalisation avantageuse du dispositif 10 permettant de réaliser la préparation d'une solution médicinale par exemple une solution injectable à partir de deux composants de base. En particulier la figure 1 représente ce dispositif pendant la phase de stockage. Il comporte un premier conteneur 11 dans lequel est stocké un lyophilisat 12, un deuxième conteneur 13 contenant dans ce cas un solvant 14, et une tige de transfert 15 pourvue d'un conduit creux 16. Le conteneur 11, appelé par la suite conteneur distal, a un fond 17 fermé et une extrémité ouverte 18 à travers laquelle a été engagé un obturateur-piston-vanne 19 qui délimite à l'intérieur du conteneur distal, une chambre 20 contenant le lyophilisat 12. On notera que ce lyophilisat 12 peut être remplacé par une autre substance active telle que par exemple un liquide, une poudre ou une substance pâteuse de viscosité plus ou moins grande. Le conteneur 13, appelé par la suite conteneur proximal, est ouvert à ses deux extrémités. Dans l'exemple représenté l'extrémité ouverte 21 est obturée par une capsule 22 fixée par sertissage. Cette capsule, connue en soi, est utilisée sur de nombreux flacons contenant des substances médicamenteuses et peut être réalisée sous la forme d'un composé de matière plastique assurant l'étanchéité et d'aluminium assurant la tenue mécanique et formant une barrière antiseptique. L'autre extrémité ouverte 23 du conteneur 13 est obturée par un obturateur-piston-vanne 24. Les deux obturateurs-pistons-vannes 19 et 24 sont pourvus chacun d'un évidement central respectivement 25, 26 dans lesquels sont respectivement engagés deux embouts 27 et 28 solidaires de la tige de transfert 15. L'obturateur-piston-vanne 24 définit avec les parois du conteneur 13 et la capsule 22 une chambre 29 contenant le solvant 14. Selon la substance médicamenteuse contenue dans la chambre 20 du conteneur distal 11, le solvant 14 peut être remplacé par un diluant. Quelle que soit la nature de la substance active du conteneur distal, la substance logée dans le conteneur proximal est toujours à l'état liquide et est prévue pour réaliser avec la substance de traitement une solution, une suspension ou une émulsion reconstituée à partir de deux composants de base et destinée à être utilisée par la suite dans le cadre d'un traitement thérapeutique sous une forme injectable administrable par voie externe ou interne au moyen d'applicateurs quelconques, notamment une aiguille, un trocart, un distributeur de gouttes, un pulvérisateur, etc..

L'obturateur-piston-vanne 24 comporte dans le prolongement de l'évidement central 26, un petit dégagement 30 dans lequel est engagée l'extrémité antérieure de la tige de transfert 15. Dans le prolongement de ce dégagement 30, l'obturateur-piston-vanne comporte une fente prédécoupée 31 qui permet précisément à cet organe de jouer le rôle de vanne fermée au stockage et ouverte pendant les autres phases, c'est-à-dire pendant l'activation et l'utilisation. Les parois en élastomère de l'obturateur-piston-vanne 24, qui entourent les lèvres de la fentes 31, sont épaisses et cette fente est ménagée dans une zone assurant une compression élevée desdites lèvres de cette fente lorsque l'obturateur-piston-vanne est en compression à l'intérieur du conteneur proximal de telle manière que l'obturation au stockage s'effectue d'une manière parfaitement efficace pour garantir à la fois l'étanchéité et la stérilité de la chambre et du contenu du conteneur proximal.

D'une manière similaire, l'obturateur-piston-vanne 19 comporte un dégagement central 32 dans lequel est engagée l'autre extrémité de la tige de transfert 15. Dans le prolongement de ce dégagement, l'obturateur-piston-vanne comporte une fente 33 qui assure la fonction de vanne fermée au stockage et ouverte pendant les autres phases d'exploitation du système c'est-à-dire l'activation et l'utilisation. Là encore l'épaisseur de la matière qui entoure les lèvres de la fente 33, prédécoupée, est suffisante lorsque l'obturateur-piston-vanne est en place à l'intérieur du conteneur distal pour assurer une compression relativement importante desdites lèvres pour garantir à la fois l'étanchéité et la stérilité de la chambre 20 du conteneur distal 11.

Dans la forme de réalisation représentée le diamètre intérieur du conteneur distal est sensiblement égal au diamètre extérieur du conteneur proximal afin qu'ils puissent être emboîtés, au moins partiellement l'un dans l'autre, au moment de l'utilisation. Pour des raisons évidentes de simplicité de fabrication et de coût de production, les deux conteneurs ont une forme générale allongée cylindrique à section circulaire. En raison des différences de diamètre des deux conteneurs l'obturateur-piston-vanne 19 a un diamètre supérieur à celui de l'obturateur-piston-vanne 24. Au moment de l'activation dont la première phase est représentée par la figure 2, initiée par l'exercice d'une pression exercée axialement de préférence sur le fond 17 du conteneur distal le conteneur proximal étant maintenu en position, les extrémités de la tige de transfert s'engagent respectivement dans les fentes 31 et 33 des obturateurs-pistons-vannes 24 et 19 ce qui a pour effet de mettre les chambres 20 et 29 en communication et d'autoriser l'écoulement du solvant 14 à travers le conduit 16 dans la chambre 20 contenant le lyophilisat 12. Le diamètre et la longueur du conduit 16 sont dimensionnés de façon que l'on obtienne un débit maîtrisé lors de l'écoulement du solvant 14 à travers ce conduit 16. On notera au passage que les extrémités de la tige de transfert ne sont pas constituées par des biseaux étant donné que leur fonction ne consiste pas à percer une membrane, mais par une section plane disposée le long de plans perpendiculaires à l'axe de la tige de transfert, et éventuellement prolongée par une zone faiblement tronconique. Cette géométrie permet une introduction aisée dans les fentes de manière étanche et stérile, l'extrémité étant destinée à écarter les lèvres de ces fentes pour ouvrir la vanne de l'obturateur-piston-vanne, contrairement à de nombreux systèmes connus où une tige de transfert présente une extrémité biseautée dont la fonction consiste à perforer une membrane. L'étanchéité entre la membrane et l'extrémité biseautée ne peut être assurée que si l'épaisseur de la membrane est nettement supérieure à la longueur du biseau, ce qui est incompatible avec une construction compacte et avec la facilité de perforer cette membrane. Par ailleurs, on notera également que la tige de transfert est pourvue d'une première butée 34 et d'une deuxième butée 35 qui sont, sauf au stockage, respectivement en appui contre les faces en regard des obturateurs-pistons-vannes 24 et 19. Ces butées ont pour fonction de définir de manière précise le degré de pénétration des extrémités de la tige de transfert dans les chambres correspondantes des conteneurs proximal et distal.

Lorsque l'utilisateur continue à appuyer sur le fond 17, tout le solvant contenu dans le conteneur proximal 13 s'écoule dans la chambre 20, entre en contact avec le lyophilisat 12 pour former une solution 12 + 14 qui constitue la substance médicinale reconstituée. Par le jeu des pressions et des différences de surfaces entre les deux obturateurs-pistons-vannes, l'obturateur-piston-vanne 24 se loge à l'extrémité frontale du conteneur proximal 13 et l'obturateur-piston-vanne 19 se met dans une position telle qu'il devient possible d'assurer le dégazage, le flacon ayant été équipé d'un embout approprié en fonction de l'utilisation prévue pour la solution médicinale. Si cette solution est destinée à être injectée, l'embout sera une aiguille. Si cette solution est destinée à être transférée dans une poche de transfusion, l'embout pourra être un trocart. Si la solution est destinée à une autre utilisation, l'embout sera choisi en conséquence. Quel que soit l'embout, la solution est transférée à travers le conduit 16. A cet effet, l'utilisateur appuie sur le fond du conteneur distal 11 qui s'emboîte sur le conteneur proximal 13. L'obturateur-piston-vanne 19 s'enfonce progressivement à l'intérieur du conteneur distal 11 jusqu'au moment où il touche le fond 17. Pour empêcher une réutilisation du dispositif, en particulier dans le cas où il est utilisé comme seringue, les moyens d'accouplement des embouts 27 et 28 de la tige de transfert 15 respectivement avec les obturateurs-pistons-vannes 19 et 24, sont suffisamment faibles pour qu'ils se désolidarisent desdits obturateurs-pistons-vannes au moment où un utilisateur tenterait de retirer le conteneur proximal du conteneur distal, rendant ainsi toute réaspiration d'un liquide impossible à l'intérieur du dispositif.

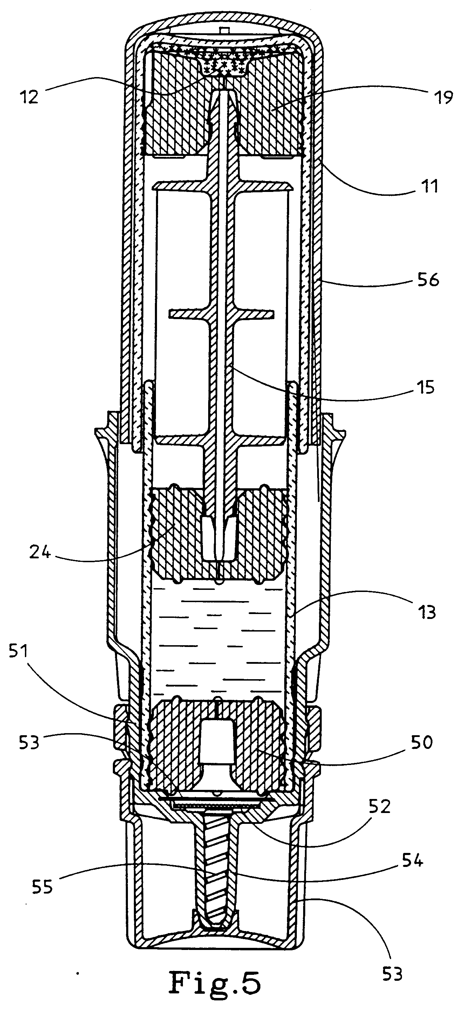

La figure 5 représente le dispositif selon l'invention adapté en vue d'une application aux traitements ophtalmiques. Dans ce cas, les deux conteneurs, respectivement distal et proximal, 11 et 13 sont préemboîtés. Le conteneur distal 11, qui contient un lyophilisat 12, est totalement vide d'air, l'obturateur-piston-vanne 19 étant engagé à l'intérieur dudit conteneur, en appui serré contre la matière qu'il contient. L'avantage de cette réalisation est qu'elle permet de supprimer totalement toute opération de purge ou de débullage et d'éviter une oxydation du mélange après sa reconstitution, c'est-à-dire au moment de l'activation du dispositif.

Le conteneur proximal 13 qui, rappelons-le, est ouvert à ses deux extrémités, est obturé à une de ses extrémités par l'obturateur-piston-vanne 24 et à son autre extrémité par un bouchon 50. Une capsule 51 coiffe ce bouchon et un filtre 52 ainsi qu'une pastille d'argent ayant une fonction oligodynamique sont interposés entre cette capsule et ce bouchon. La pastille d'argent peut être remplacée par un autre matériau tel que par exemple de l'oxyde d'argent ou un composé contenant des substances ayant des propriétés similaires. Un capuchon de protection 53 ferme cet ensemble. Un embout 54 à canule spirale 55, équipe l'extrémité de la capsule 51. Dans ce cas, la tige de transfert 15 est avantageusement constituée d'un élément en matière synthétique rigide moulé d'une pièce. On notera que le bouchon 50 présente une géométrie qui est sensiblement identique à celle de l'obturateur-piston-vanne 24 et qu'il fonctionne sur les mêmes principes, du moins en ce qui concerne son ouverture centrale qui en fait une vanne constituée d'une fente prédécoupée destinée à être ouverte par l'extrémité de la tige de transfert, dans la phase d' utilisation du dispositif. Dans cette réalisation également, la capsule 51 a une jupe suffisamment longue pour recouvrir une partie du conteneur distal au voisinage de son extrémité ouverte. Il se trouve que ce conteneur distal 11 est lui-même logé à l'intérieur d'un capuchon 56 en matière synthétique moulée qui assure sa protection. Le capuchon 56 est pré-emboîté dans la jupe de la capsule 51. Pour d'autres utilisations, le capuchon 56 pourrait être supprimé et le conteneur distal pourrait être directement engagé, autour de sa partie supérieure ouverte, à l'intérieur de la jupe de la capsule 51.

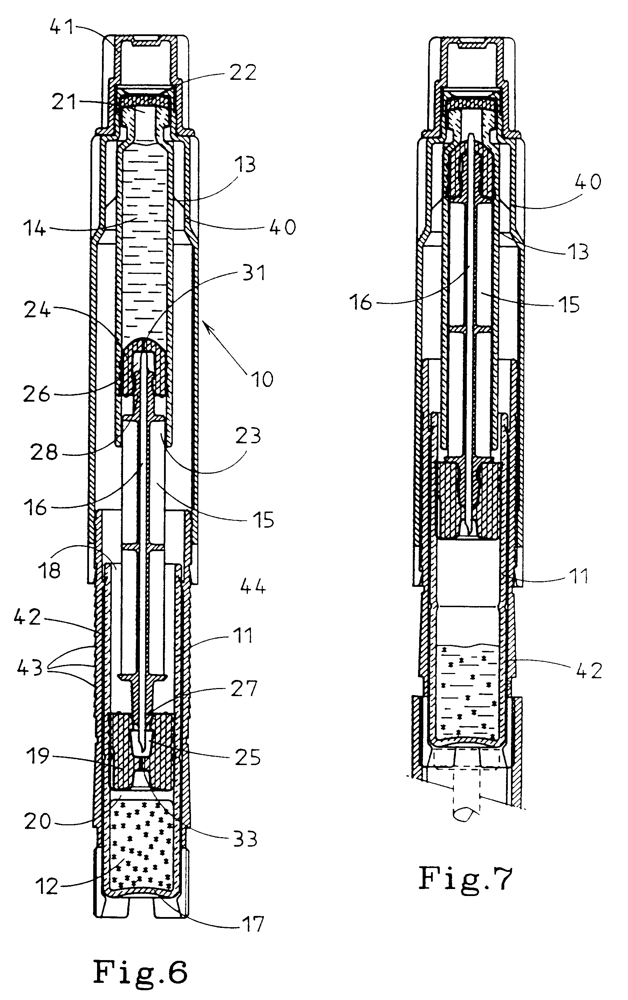

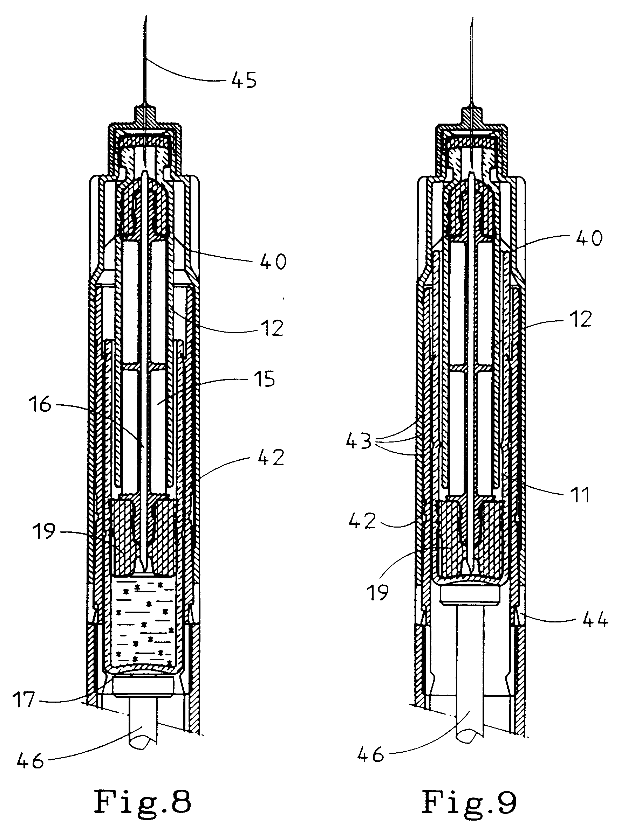

Les figures 6 à 9 illustrent une autre forme de réalisation du dispositif destiné à être utilisé comme une seringue en vue de l'injection de la substance médicinale reconstituée. La figure 6 représente le stockage, la figure 7 l'activation, les figures 8 et 9 l'utilisation dans le cadre d'un système mécanique à multidoses. Comme précédemment, le dispositif 10 comporte un conteneur distal 11 contenant par exemple un lyophilisat 12 et un conteneur proximal 13 contenant par exemple un solvant 14, ainsi qu'une tige de transfert 15 destinée à permettre la communication entre ces deux conteneurs à travers un conduit tubulaire 16. Le conteneur distal 11 a un fond fermé 17 et présente une ouverture 18 à son extrémité opposée au fond 17. Il est obturé par un obturateur-piston-vanne 19 qui délimite une chambre 20 contenant le lyophilisat 12. Le conteneur proximal 13 présente une ouverture supérieure 21 obturée par une capsule sertie 22. Comme ce conteneur est ouvert à ses deux extrémités, son autre extrémité 23 est obturée par un obturateur-piston-vanne 24. L'obturateur-piston-vanne 19 comporte un évidement central 25 et l'obturateur-piston-vanne 24 comporte un évidement central 26, ces deux obturateurs étant destinés à recevoir respectivement deux embouts d'extrémité 27 et 28 de la tige de transfert 15. L'obturateur-piston-vanne 24 comporte une fente prédécoupée 31 qui fait office de vanne et qui est destinée à recevoir l'extrémité de la tige de transfert 15 et l'obturateur-piston-vanne 19 comporte également une fente prédécoupée 33 faisant office de vanne qui est destinée à être traversée par l'autre extrémité de la tige de transfert 15.

On constate qu'à quelques détails de construction près, les éléments décrits ci-dessus sont identiques à ceux décrits en référence aux figures 1 à 4. Toutefois, on notera que le conteneur proximal 13 est logé à l'intérieur d'un premier élément tubulaire 40 dont l'extrémité proximale est coiffée d'un capuchon protecteur 41 et que le conteneur distal 11 est logé à l'intérieur d'un second élément tubulaire 42 partiellement engagé à l'intérieur du premier élément tubulaire 40. Dans la position de stockage représentée par la figure 6, les deux éléments tubulaires 40 et 42 définissent une boîte sensiblement cylindrique à l'intérieur de laquelle est logé le dispositif 10 tel que représenté par la figure 1. En vue de l'activation de ce dispositif, le second élément 42 comporte une série de crans 43 qui peuvent être disposés sur toute la périphérie de l'élément ou uniquement sur une zone déterminée de cette périphérie. Ces crans sont destinés à coopérer avec au moins une languette flexible 44 ménagée à l'intérieur de la paroi latérale de l'élément 40 en vue de générer des forces de freinage prédéterminées destinées à réguler le déplacement axial relatif des deux éléments 40 et 42 et en vue de définir des butées anti-retour empêchant le recul ou le retrait du deuxième élément 42 lorsqu'il est engagé à l'intérieur du premier élément 40.

La figure 7 représente la phase d' activation et de préparation du mélange des deux composants. Dans cette phase, l'élément tubulaire 42 a été enfoncé à l'intérieur de l'élément tubulaire 40, ce qui a pour effet de déplacer le conteneur distal en direction du conteneur proximal. Il en résulte d'une part la pénétration des deux extrémités de la tige de transfert dans les fentes constituant les vannes des obturateurs-pistons-vannes correspondants, le déplacement de l'obturateur-piston-vanne 24 axialement à l'intérieur du conteneur proximal et l'écoulement du solvant dans la chambre 20 où se trouve le lyophilisat 12. A la fin de cette phase d'activation, le mélange des deux substances ou constituants de base de la substance médicinale est terminé et cette substance est prête à l'emploi.

La figure 8 illustre la dernière phase de préparation qui consiste à dégazer ou débuller le dispositif qui, dans ce cas, est équipé d'une aiguille 45 en vue de l'injection de la substance médicinale reconstituée. Le second élément tubulaire 42 a été complètement repoussé à l'intérieur du premier élément tubulaire 40, le conteneur distal dans lequel s'est effectué la dissolution du lyophilisat a été entièrement dégazé, et une poussée exercée sur le fond 17 dudit conteneur distal au moyen d'un poussoir approprié 46 permet de faire pénétrer l'obturateur-piston-vanne 19 en appui contre la solution contenue dans ledit conteneur distal. Par la suite, cet obturateur-piston-vanne a une fonction de piston et refoule la solution à travers le conduit 16 de la tige de transfert 15 en direction de l'aiguille d'injection 45.

A la fin de la phase d' utilisation illustrée par la figure 9, le poussoir 46 a repoussé intégralement le conteneur distal en direction de l'extrémité proximale du dispositif de telle manière que l'obturateur-piston-vanne 19 a été refoulé complètement à l'intérieur du conteneur distal et que la quasi totalité de la substance injectable constituée par la solution de substance médicinale reconstituée a été évacuée. Par ailleurs, on notera que le second élément cylindrique 42 est complètement emboîté dans le premier élément cylindrique 40 et que le ou les crans 43 coopèrent avec la languette flexible 44 pour bloquer les deux éléments l'un par rapport a l'autre et empêcher tout retour du second élément dans sa position initiale. Grâce à ces organes, le dispositif est garanti non réutilisable.

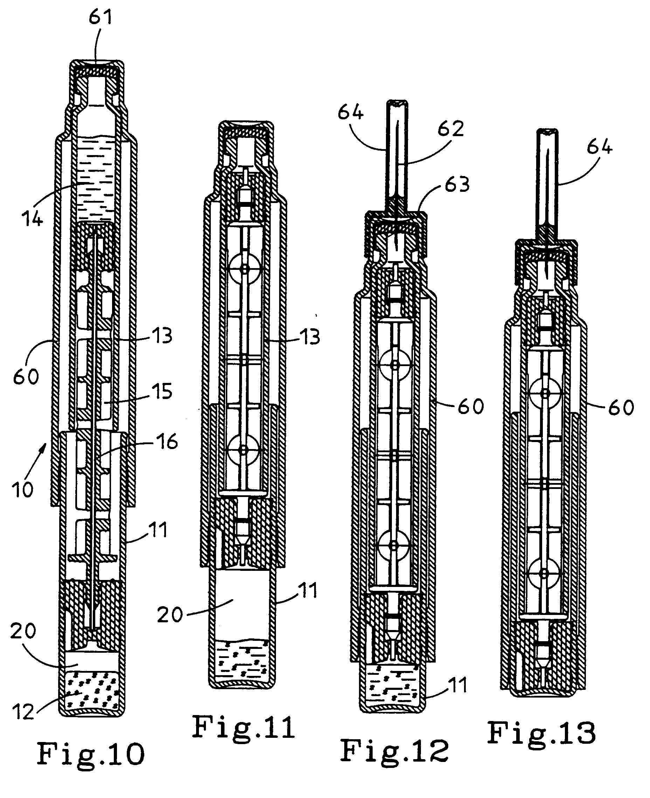

Les figures 10 à 13 représentent une variante qui peut être considérée comme un perfectionnement du dispositif tel que représenté par les figures 1 à 4 et correspondant en fait à ce que l'on pourrait appeler le réservoir primaire. Le dispositif tel que représenté par la figure 10 à l'état stocké correspond sensiblement à l'association dudit réservoir primaire tel que représenté par la figure 1 avec son conteneur distal 11, son conteneur proximal 13 et sa tige de transfert 15, logés au moins partiellement, du moins au stockage, à l'intérieur d'un corps cylindrique ou manchon 60. Ce manchon est de préférence réalisé en matière synthétique moulée. Il comporte a son extrémité antérieure une zone de moindre épaisseur 61 pouvant être aisément perforée par une aiguille rapportée comme le montre la figure 12. Par ailleurs, on notera que la tige de transfert contient le conduit intérieur 16 qui est de préférence constitué par une canule creuse en métal qui est surmoulée.

La figure 11 représente le dispositif 10 en phase terminale d'activation, le solvant 14 ayant été transféré à travers la tige de transfert 15 dans la chambre 20 du conteneur distal 11 pour dissoudre le lyophilisat 12.

La figure 12 illustre la phase de dégazage de la seringue obtenue par adjonction d'une aiguille 62 prémontée sur une embase 63 et protégée par un capuchon de protection 64. On notera que le conteneur distal 11 a été repoussé en direction de l'extrémité proximale du dispositif afin d' évacuer l'air contenu dans la chambre 20 dudit réservoir distal après réalisation du mélange.

La figure 13 représente le dispositif en fin d' utilisation. On notera que le capuchon 64 a été repositionné à l'extrémité du dispositif pour masquer l'aiguille et éviter au personnel soignant une blessure accidentelle par cet outil piquant. Enfin, les deux conteneurs distal et proximal sont emboîtés l'un dans l'autre et complètement logés à l'intérieur du manchon 60, ce qui empêche toute réutilisation de la seringue étant donné qu'il est impossible, sans casser ce manchon, de tirer le conteneur distal vers l'arrière. Même si cette opération pouvait être réalisée, la sécurité obtenue grâce à une désolidarisation effective des obturateurs-pistons-vannes et de la tige de transfert reste acquise, comme cela a été expliqué en référence à la description des figures 1 à 4.

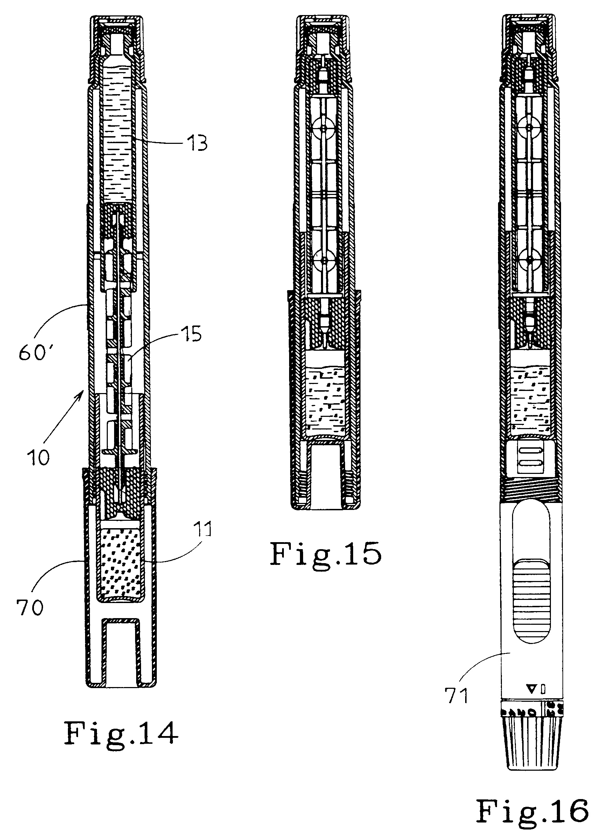

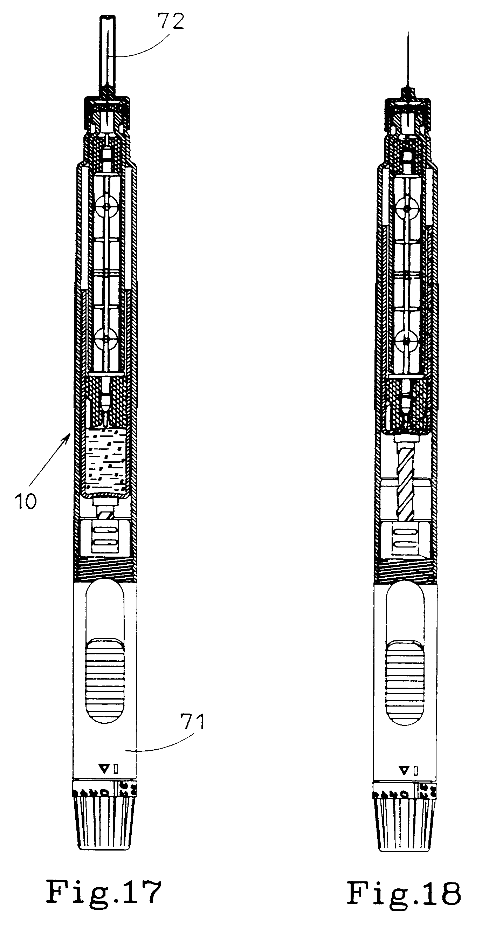

Les figures 14 à 18 illustrent une autre forme de réalisation, qui dérive de la réalisation précédente, dans laquelle le manchon 60 a été remplacé par un manchon 60' plus long que le manchon 60. A ce manchon 60' est adjoint un capuchon poussoir 70 ainsi qu'un mécanisme de dosage 71, connu en soi et commercialisé par exemple sous la dénomination "PEN" ou similaire. Le dispositif 10 comporte comme précédemment les éléments principaux suivants qui sont le conteneur distal 11, le conteneur proximal 13 et la tige de transfert 15. Il est représenté par la figure 14 à l'état de stockage. La figure 15 représente l'état activé. La figure 16 représente le dispositif activé et équipé du mécanisme de dosage 71. La figure 17 représente le dispositif activé équipé du mécanisme de dosage et pourvu d'une seringue d' injection 72. La figure 18 représente le dispositif en fin d'utilisation.

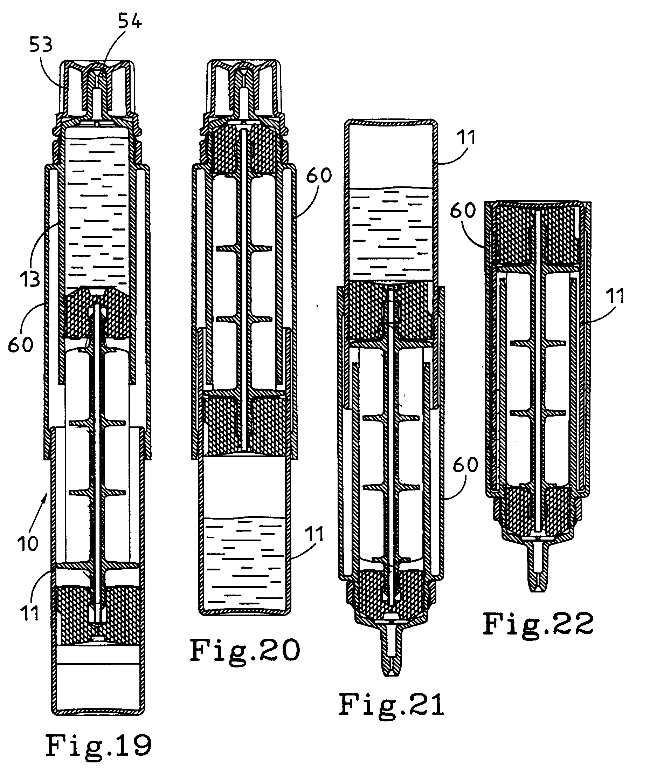

Les figures 19 à 22 illustrent le dispositif adapté en vue d'une application au domaine ophtalmique. On notera que dans cette réalisation le conteneur proximal 13 est réalisé en matière synthétique moulée d'une pièce avec le manchon 60 qui contient intégralement ledit conteneur proximal et partiellement, au moins au stockage, le conteneur distal 11. Le manchon 60 se prolonge par un applicateur de gouttes ophtalmiques constitué par un embout 54 sensiblement identique, du moins dans sa fonction, à celui décrit en référence à la figure 5. Cet embout pourrait également se présenter sous la forme d'un pulvérisateur nasal. Un capuchon de protection 53 assure une fermeture étanche et stérile du dispositif pendant la phase de stockage représentée par la figure 19 et la phase d' activation représentée par la figure 20. La phase d'utilisation est représentée par la figure 21. La fin de l'utilisation est représentée par la figure 22. On notera que le conteneur distal 11 est intégralement enfoncé à l'intérieur du manchon 60, ce qui garantit la non-réutilisation du dispositif.

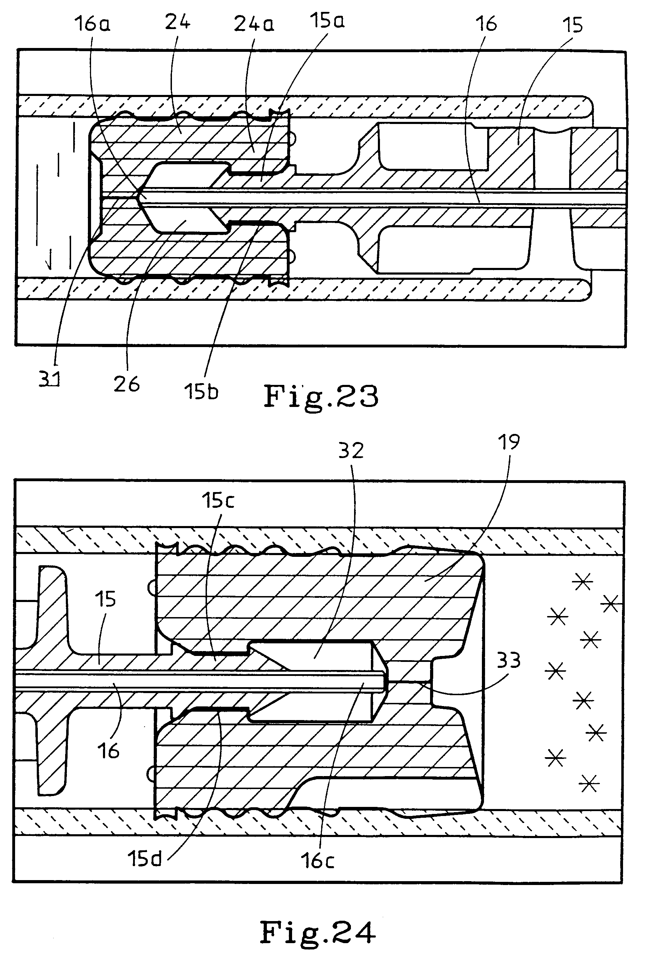

La figure 23 représente une vue agrandie de l'obturateur-piston-vanne 24 et de l'extrémité correspondante de la tige de transfert équipée de son conduit central dans la position de stockage. Dans cette position, l'extrémité 15a de la tige de transfert proprement dite est engagée dans une première zone 24a de l'obturateur-piston-vanne 24, cet engagement étant réalisé de manière à garantir une parfaite étanchéité et une barrière stérile grâce à une compression élastique qui s'exerce au niveau de la surface 15b. Dans cette position également, l'extrémité 16a du conduit 16, qui est délimitée par une section plane sensiblement perpendiculaire à l'axe de ce conduit, est en appui contre l'entrée de la fente 31 qui constitue la vanne de l'obturateur-piston-vanne 24. Comme mentionné précédemment, cette vanne est maintenue fermée de manière étanche par la compression de la matière relativement importante qui entoure ses lèvres. L'ouverture de cette vanne ne s'effectue pas, comme dans de nombreux systèmes antérieurs, sous l'effet de la pression exercée par une substance liquide qui est forcée à travers le conduit 16 mais sous la pression mécanique d'introduction de l'extrémité 16a de ce conduit. De ce fait, l'étanchéité entre les parois du conduit 16 et les lèvres de la fente 31 reste préservée et l'évidement central 26 de l'obturateur-piston-vanne 24 n'entre jamais en contact avec les composants des deux conteneurs ni avec la substance reconstituée.

La figure 24 représente une vue agrandie similaire à celle de la figure 23, mais concernant l'obturateur-piston-vanne 19 à l'état de stockage. Comme précédemment, l'autre extrémité 15c de la tige de transfert 15 est engagée dans un évidement central 32 de l'obturateur-piston-vanne 19 et l'extrémité 16c du conduit est en appui contre l'entrée de la fente 33 faisant office de vanne. L'étanchéité et la stérilité sont garanties au niveau de la surface 15d grâce à la compression exercée par la matière de l'obturateur-piston-vanne 19 sur l'extrémité 15c de la tige de transfert dans la zone concernée. De même, l' étanchéité entre l'extrémité 16c du conduit 16 et les lèvres de la fente 33 est assurée lorsque cette extrémité est engagée dans ladite fente.

On notera par ailleurs que pour les deux obturateurs-pistons-vannes 19 et 24, respectivement représentés par les figures 24 et 23, les extrémités 15a d'une part et 15c d'autre part de la tige de transfert 15 présentent une forme de harpon pourvu de butées d'arrêt garantissant une connexion relativement ferme de la tige de transfert et des obturateurs-pistons-vannes pendant la phase de stockage. Grâce à cette forme de harpon, ces extrémités 15a et 15c définissent des butées d'arrêt et des moyens de liaison qui permettent de définir une position extrêmement précise desdits obturateurs-pistons-vannes à l'intérieur du dispositif.

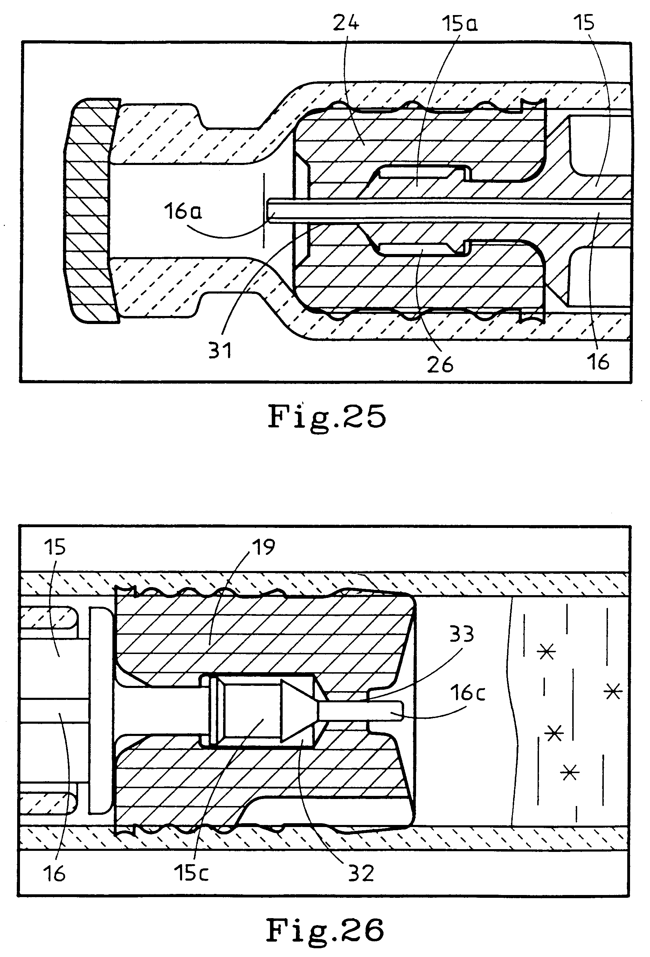

La figure 25 représente l'obturateur-piston-vanne 24 de la figure 23 dans la position d'activation du dispositif. L'extrémité 15a de la tige de transfert a été repoussée à l'intérieur de l'évidement central 26 et l'extrémité 16a du conduit 16 a été repoussée à travers la fente prédécoupée 31. Cette figure montre bien que les zones d' étanchéité et de stérilité mentionnées précédemment sont préservées dans cette phase.

La figure 26 représente l'obturateur-piston-vanne 19 représenté par la figure 24, dans la phase d'activation. Là encore, l'extrémité 15c de la tige de transfert 15 a été repoussée à l'intérieur de l'évidement central 32 et l'extrémité 16c du conduit 16 a été poussée à travers la fente prédécoupée 33. Comme précédemment, les zones d' étanchéité et les barrières de stérilité sont préservées dans cette phase.

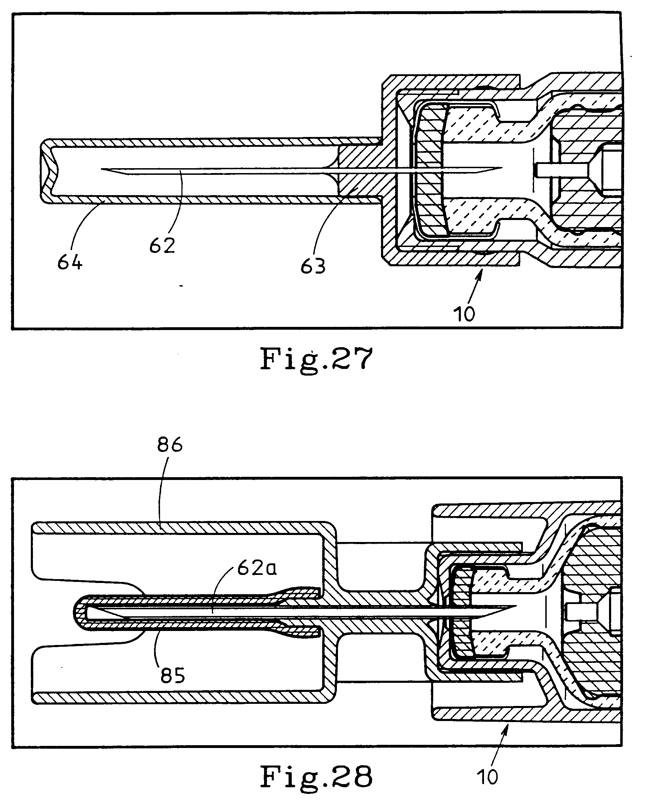

Les figures 27 à 30 illustrent différents accessoires ou périphériques destinés à être associés au dispositif 10 en vue d'utilisations spécifiques de ce dispositif. En particulier, la figure 27 est une vue agrandie de l'extrémité du dispositif 10 tel que représenté par la figure 12, l'aiguille 62 montée sur une embase 63 est protégée par un capuchon de protection 64.

La figure 28 représente une autre forme de réalisation dans laquelle une aiguille de transfert 62a est protégée par une garde 80 rigide en matière synthétique, cette aiguille 62a étant par ailleurs recouverte d'un manchon souple 81. Ce manchon 81 a pour fonction d'éviter une nébulisation de la substance reconstituée à l'intérieur du dispositif 10 au moment de la connexion de ce dispositif à un autre conteneur tel que par exemple une poche de transfusion, à travers l'aiguille de transfert 62a. En effet, en raison d'une légère surpression régnant à l'intérieur du dispositif 10, il pourrait se produire une nébulisation de la substance médicinale, qui peut être dangereuse pour le personnel soignant. Cette nébulisation est évitée grâce à la protection assurée par le manchon 81 qui est souple et se replie sur lui-même au fur et à mesure que l'aiguille de transfert est enfoncée dans un embout approprié de la poche de transfusion, puis se déplie lors du retrait et de la déconnexion pour obturer le dispositif 10.

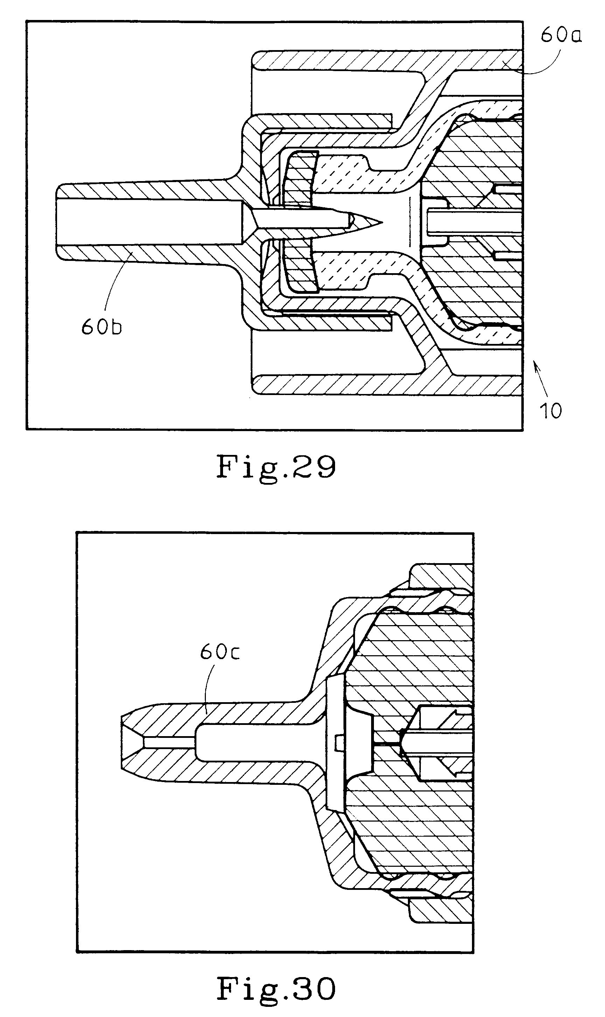

La figure 29 représente le dispositif 10 pourvu d'un manchon 60a ayant sensiblement les mêmes fonctions que le manchon 60 représenté par la figure 10. Sur l'extrémité de ce manchon est adapté un embout 60b ayant la forme d'un cône Luer femelle permettant d'assurer le raccordement d'accessoires tels qu'un support porte-aiguille, un embout de connexion Luer mâle, etc..

La figure 30 est similaire à la figure 29 mais l'embout 60b a été remplacé par un embout 60c en forme de cône Luer mâle.

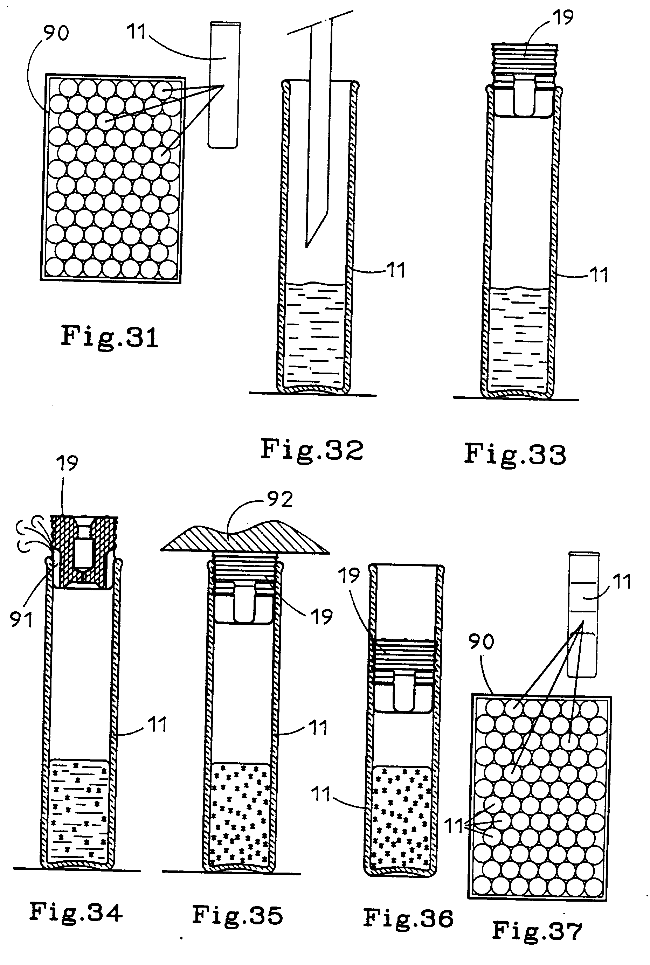

Les figures suivantes représentent schématiquement les phases cinématiques d'assemblage et de construction du dispositif 10 et de ses composants dans les différents cas de figures, c est-à-dire lorsque le conteneur distal est destiné à contenir un lyophilisat, un liquide plus ou moins pâteux et une poudre. La figure 31 montre le conteneur distal 11 rangé dans une cassette 90. On notera que ce rangement serré est possible grâce à la forme cylindrique de ce conteneur. La figure 32 illustre le remplissage du conteneur distal 11 au moyen d'une solution destinée à être lyophilisée.

La figure 33 illustre une phase de prépositionnement de l'obturateur-piston-vanne 19.

La figure 34 illustre la phase de lyophilisation pendant laquelle la vapeur et les gaz peuvent s'échapper par des rainures 91 ménagées sur la zone inférieure de l'obturateur-piston-vanne 19. On notera que la transmission des énergies est aisée grâce au fond plat du flacon ce qui favorise la formation homogène des cristaux de glace et la dessiccation quasi totale du produit lyophilisé.

La figure 35 illustre la phase de fermeture étanche du conteneur distal. Cette fermeture s'effectue par l'enfoncement de l'obturateur-piston-vanne 19 au moyen d'un poussoir 92 qui peut être constitué directement par le plafond de la chambre de lyophilisation à l'intérieur de laquelle s'effectue l'opération.

La figure 36 illustre une phase de positionnement de l' obturateur-piston-vanne 19, positionnement qui se fait automatiquement par aspiration de cet organe à l'intérieur du conteneur distal dans lequel règne le vide, puis mécaniquement pour ajuster la position finale.

La figure 37 illustre le retour des conteneurs remplis dans le magasin 90.

On notera que toutes ces opérations s'effectuent avec des moyens standards largement utilisés dans l'industrie pharmaceutique.

Dans le cas où la substance active contenue dans le conteneur 11 est un liquide et non un lyophilisat, le processus est simplifié puisqu'il ne comporte que les phases de remplissage représentée par la figure 32, de prépositionnement de l'obturateur-piston-vanne représentée par la figure 33 et de mise en place finale de cet obturateur-piston-vanne, éventuellement au moyen d'un poussoir approprié qui permet un dégazage à l'intérieur du conteneur.

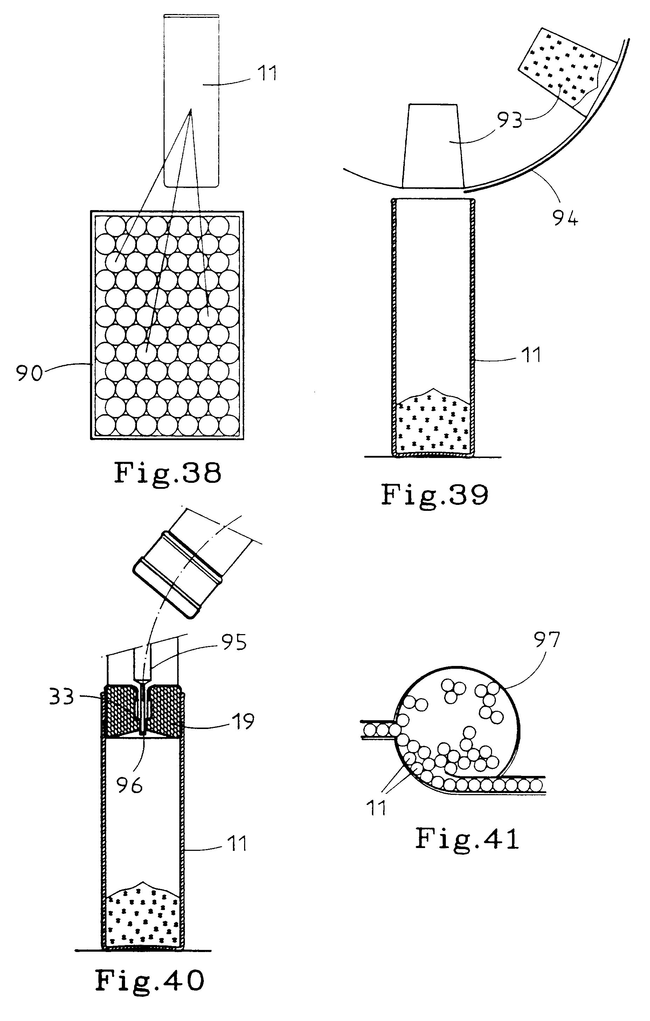

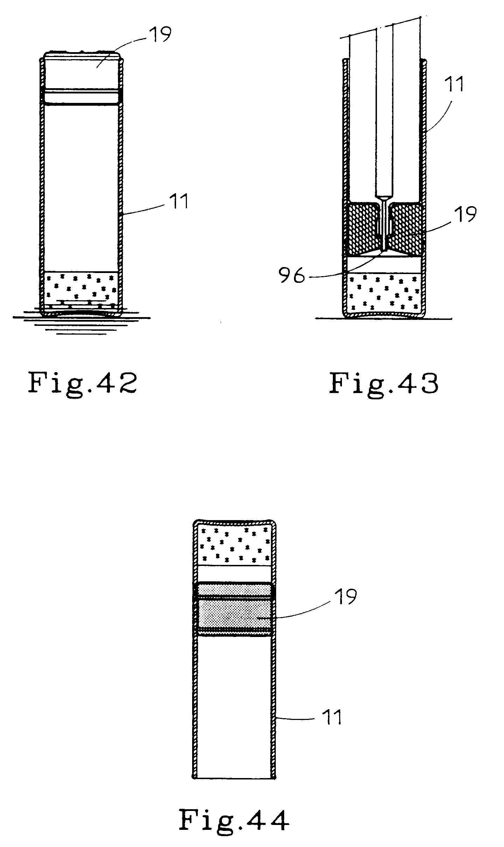

Les figures 38 à 44 illustrent le remplissage du conteneur distal 11 au moyen d'une poudre. En particulier, la figure 38 montre les conteneurs 11 logés dans une cassette 90. La figure 39 illustre le remplissage du conteneur 11 au moyen d'une dose de poudre, ces doses étant contenues dans des godets 93 montés sur un système d'amenée du type à roue 94, utilisé dans l'industrie pharmaceutique. La figure 40 illustre une phase de prépositionnement de l'obturateur-piston-vanne 19, accompagnée d'une phase de dégazage au moyen d'un embout d'aspiration 95 de forme appropriée. Cet embout comporte à cet effet une canule 96 qui est engagée à travers la fente 33 dudit obturateur-piston-vanne. Après retrait de cet instrument, les conteneurs distaux sont introduits en vrac sur un plateau de transfert 97 d'où ils ressortent selon une disposition ordrée en vue de traitements ultérieurs comme le montre la figure 41. La figure 42 représente le conteneur distal 11 avec son obturateur-piston-vanne 19 prépositionné tel qu'il ressort du plateau de transfert 97 de la figure 41. Cette phase de vibration de la poudre est nécessaire pour assurer son tassement au fond du conteneur. Le positionnement précis de l'obturateur-piston-vanne 19 à l'intérieur du conteneur distal 11 s'accompagne, comme le montre la figure 43, d'une aspiration des gaz surmontant la poudre à travers la canule 96 décrite en référence à la figure 40. La figure 44 illustre le conteneur distal 11 avec l'obturateur-piston-vanne positionné convenablement. La poudre est raclée des parois du conteneur lors de la mise en place de cet obturateur-piston-vanne grâce à la pression qu'il exerce sur ces parois. Néanmoins, une phase de décontamination consistant à évacuer les résidus de poudre sur les parois intérieure et extérieure du conteneur distal reste nécessaire.

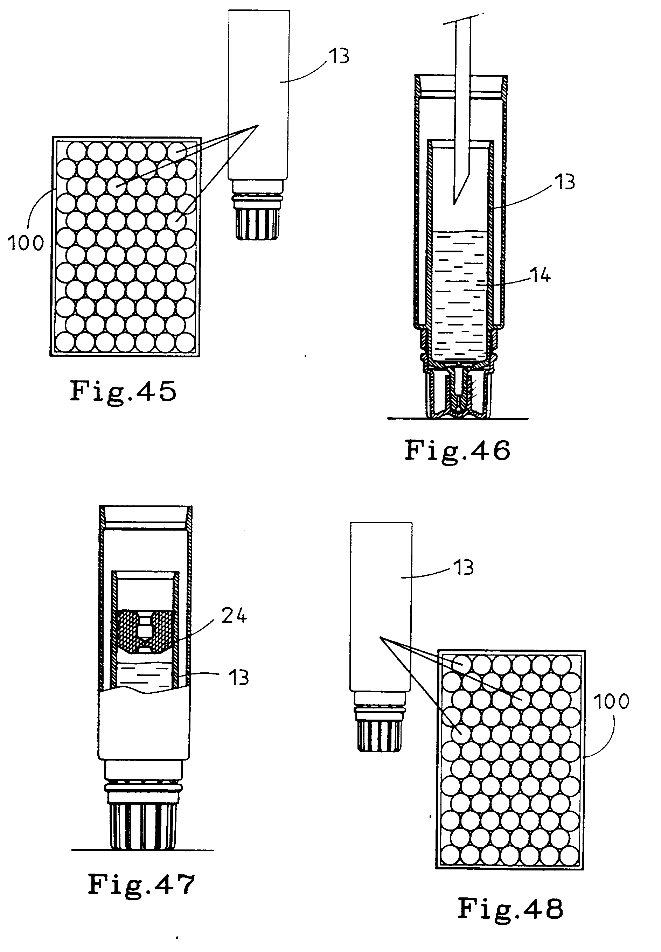

Les figure 45 à 54 illustrent l'assemblage du dispositif 10 plus spécifiquement destiné à une application ophtalmique. La figure 45 illustre le conteneur proximal 13 logé dans une cassette 100. Ce conteneur correspond à ceux qui ont été décrits précédemment en référence notamment à la figure 5 et aux figures 19 à 22. La figure 46 illustre la phase de remplissage de ce conteneur 13 au moyen d'un liquide 14 qui peut être un solvant ou un diluant, comme cela a été mentionné précédemment. La figure 47 illustre la phase de mise en place de l'obturateur-piston-vanne 24 à l'intérieur du conteneur 13. Après ces différentes interventions, les conteneurs 13 sont replacés dans des cassettes 100 (voir figure 48). Comme précédemment, ce rangement serré en cassette n'est possible que grâce au fait que ces conteneurs ont une forme cylindrique dépourvue d'aspérités et notamment d'ailettes protubérantes.

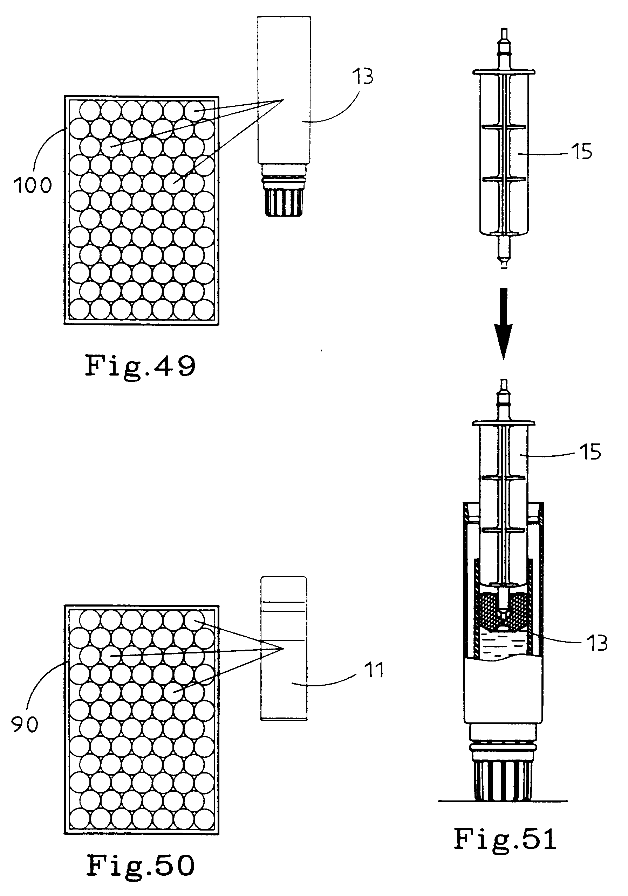

Les conteneurs 13 disposés en cassette peuvent être stockés pendant une durée plus ou moins longue et éventuellement soumis à des traitement tels que par exemple une stérilisation. Cette phase est illustrée par la figure 49. La figure 50 représente une cassette 90 dans laquelle sont placés les conteneurs distaux 11. La figure 51 illustre la mise en place de la tige de transfert 15 dans le conteneur proximal 13.

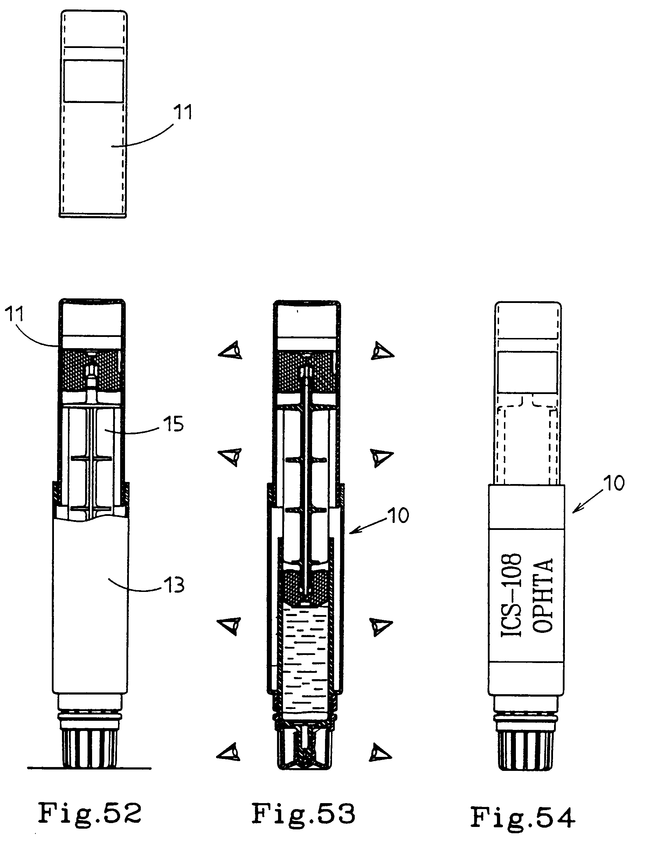

La figure 52 illustre la phase d'assemblage terminale qui consiste à mettre en place le conteneur distal 11 sur la tige de transfert 15 préalablement positionnée à l'intérieur du conteneur proximal 13. La figure 53 illustre une phase de contrôle visuel du dispositif 10 prémonté. La figure 54 illustre le dispositif 10 entièrement assemblé et contrôlé suite aux différentes interventions précisées ci-dessus.

On notera que toutes ces opérations peuvent être réalisées dans des équipements standards largement utilisés dans l'industrie pharmaceutique et ne nécessitent aucune machine spéciale pour l'assemblage des différents composants du dispositif. Pour faciliter cet assemblage et en diminuer le coût, le nombre des changements de modes de rangement, notamment le nombre de passages d'une disposition ordonnée vers une disposition désordonnée et réciproquement, est limité au minimum.

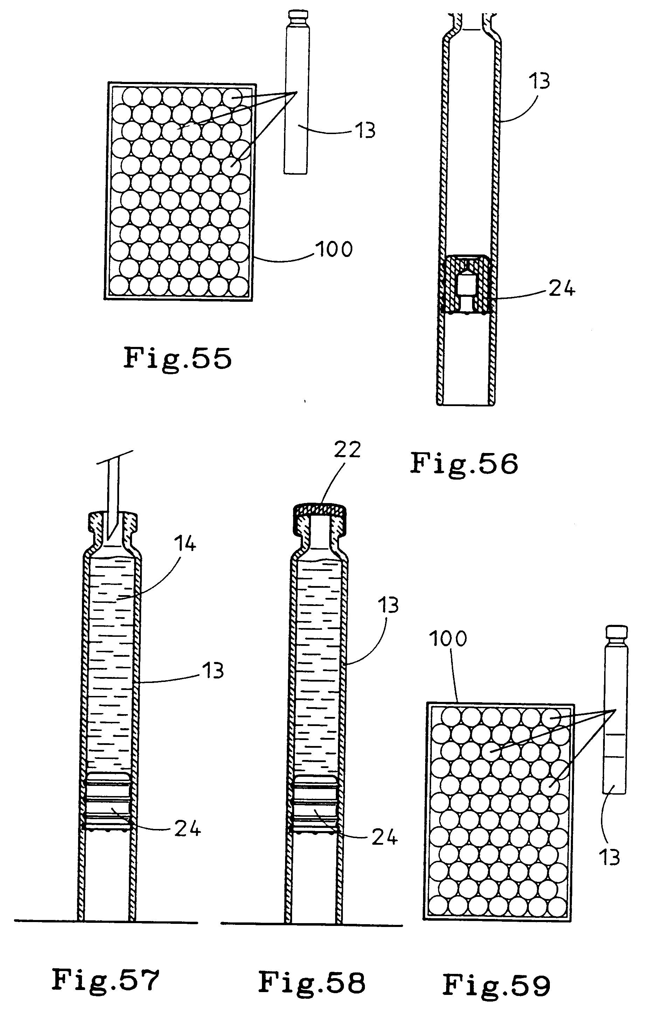

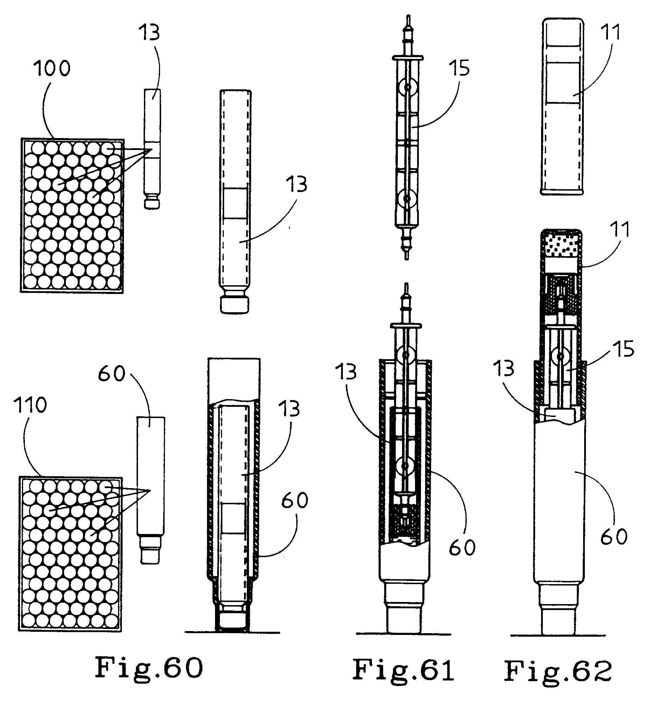

Les figures 55 à 62 illustrent le mode de préparation du conteneur proximal 13 et la mise en place de la tige de transfert 15. Le conteneur vide 13 est, comme le montre la figure 55, logé dans des cassettes 100. La figure 56 illustre la phase de positionnement de l'obturateur-piston-vanne 24 à l'intérieur de ce conteneur 13. La figure 57 illustre la phase de remplissage du solvant ou du diluant 14. La figure 58 illustre l'encapsulage de ce conteneur, c est-à-dire la mise en place de la capsule 22. La figure 59 illustre le retour des conteneurs 13 dans les cassettes 100 en vue de leur stockage momentané ou de traitements ultérieurs tels que par exemple une stérilisation. La figure 60 illustre la mise en place des conteneurs 13 tels qu'ils sont stockés dans les cassettes 100 après les éventuels traitements de stérilisation ou similaires, à l'intérieur d'un manchon 60 tel que représenté par la figure 10. Ces manchons 60 sont euxmêmes prélevés à l'intérieur de cassettes 110 où ils ont été stockés en vue de cet assemblage. La figure 61 représente la mise en place de la tige de transfert 15 sur le conteneur proximal 13 préalablement monté à l'intérieur du manchon 60. La figure 62 représente la mise en place du conteneur distal 11 sur l'autre extrémité de la tige de transfert 15 préalablement assemblée comme le montre la figure 61.

Là encore on peut affirmer que l'assemblage se fait avec des moyens standards qui sont utilisés couramment dans l'industrie pharmaceutique et qui ne nécessitent pas d'équipements spéciaux. En outre, la plupart des composants et notamment les conteneurs distal et proximal ainsi que le manchon dans lequel est disposé le conteneur proximal sont logés dans des cassettes ce qui réduit ou supprime le nombre de passages d'un état ordonné vers un état désordonné.

## Revendications

1. Dispositif pour la préparation d'une solution, d'une suspension ou d'une émulsion d'une substance médicinale active, à partir de deux composants dont l'un est à l'état de poudre, de lyophilisat, de liquide ou de pâte et dont l'autre est un diluant ou un solvant liquide, et qui sont respectivement stockés dans deux conteneurs allongés, soit un conteneur distal (11) à fond (17) fermé et un conteneur proximal (13), qui présentent chacun, à une de leurs extrémités, une ouverture obturée par un obturateur-piston-vanne, mobile axialement dans le conteneur correspondant, ces deux obturateurs-pistons-vannes (19, 24) étant reliés par une tige de transfert (15) cette tige de transfert étant agencée pour assurer une liaison de couplage rigide, étanche et stérile entre les deux obturateurs-pistons-vannes (19, 24) lorsque le dispositif (10) se trouve dans une première position dite position de stockage, et pour définir un canal de communication (16) étanche et stérile entre les deux conteneurs (11, 13) lorsque le dispositif se trouve dans une deuxième position dite position d'utilisation, les deux obturateurs-pistons-vannes (19, 24) étant pourvus respectivement, d'un évidement central (25, 26), dans lequel est engagé un embout (27, 28) solidaire de la tige de transfert (15), cette tige de transfert étant pourvue d'une première butée (34) et d'une deuxième butée (35) qui sont, sauf au stockage, respectivement en appui contre les faces en regard des obturateurs-pistons-vannes (24) et (19), et dans lequel le conteneur distal (11) contient ledit composent à l'état de poudre, de lyophilisat, de liquide ou de pâte et le conteneur proximal (13) contient ledit diluant ou solvant liquide (14), dans lequel le conteneur distal a une section transversale supérieure à celle du conteneur proximal, l'obturateur-piston-vanne (19) du conteneur distal (11) ayant une section transversale supérieure à celle de l'obturateur-piston-vanne (24) du conteneur proximal (13), dans lequel les extrémités de la tige de transfert (15) sont coupées selon un plan sensiblement perpendiculaire à son axe longitudinal, et dans lequel ces obturateurs-pistons-vannes ont une zone centrale pourvue d'une fente (31, 33) fortement comprimée et fermée de façon étanche lorsque le dispositif est en position de stockage, et ouverte lorsqu'une extrémité de la tige de transfert (15) y est introduite, le dispositif étant dans ladite deuxième position.

2. Dispositif selon la revendication 1, caractérisé en ce qu'il comporte un manchon (60) dans lequel est logé le conteneur proximal (13) et au moins partiellement le conteneur distal (11).

3. Dispositif selon la revendication 2, caractérisé en ce que le manchon (60) est associé à un capuchon poussoir (70) et à un mécanisme de dosage (71) pour l'application de doses multiples.

4. Dispositif selon la revendication 2, caractérisé en ce que ledit manchon (60) est réalisé d'une pièce par moulage avec le conteneur proximal (13).

5. Dispositif selon la revendication 4, caractérisé en ce que ledit manchon (60) est pourvu d'un embout (54) constituant un applicateur de gouttes ophtalmiques ou un pulvérisateur nasal, intégré au conteneur proximal (13).

6. Dispositif selon la revendication 2, caractérisé en ce que ledit manchon se compose d'un premier élément tubulaire (40) et d'un second élément tubulaire (42) emboîtables l'un dans l'autre, le premier élément tubulaire contenant ledit conteneur proximal (13) et ledit second élément secondaire contenant ledit conteneur distal (11).

7. Dispositif selon la revendication 6, caractérisé en ce que ledit premier élément tubulaire comporte des crans (43) et en ce que ledit second élément tubulaire comporte au moins deux languettes (44) agencées pour coopérer avec lesdits crans.

8. Dispositif selon la revendication 1, caractérisé en ce que lesdits obturateurs-pistons-vannes (19, 24) sont agencés pour assurer une fermeture étanche et stérile des conteneurs (11, 13) correspondants, par appui contre les parois intérieures de ces conteneurs, de telle manière que ces conteneurs peuvent être stockés et traités indépendamment.

## Claims

1. Device for the preparation of a solution, suspension or emulsion from an active medicinal substance by using two components, one of which is in powder form, a lyophilisate, a liquid or a paste, and the second is a dilutant or a liquid solvent, and which respectively are stored in two elongated containers, the one container being a rear container (11), closed at the bottom (17), and the other container a front container (13), each of which has at one of its ends an opening closed by a stopper-piston-valve, axially movable in the corresponding container, these two stopper-piston-valves (19, 24) being connected together by a transfer shaft (15), this transfer shaft being arranged to ensure a rigid, tight and sterile coupling between the two stopper-piston-valves (19, 24) when the device (10) is in a first position, namely a storage position, and to define a channel (16) of tight and sterile communication between the two containers (11, 13) when the device is in a second position, namely the use position, the two stopper-piston-valves (19, 24) being respectively provided with a central groove (25, 26) in which a ferrule (27, 28) integral with the transfer shaft (15) is fitted, this transfer shaft being equipped with a first stop (34) and a second stop (35) which, except for storage, are respectively in support of the surfaces in regard to stopper-piston-valves (24) and (19), and in which the rear container (11) contains said component in powder form, as a lyophilisate, as a liquid or a paste, and the front container (13) contains said dilutant or liquid solvent (14), in which the rear container has a transverse section which is greater than that of the front container, the stopper-piston-valve (19) of the rear container (11) having a transverse section which is greater than that of the stopper-piston-valve (24) of the front container (13), in which the ends of the transfer shaft (15) are shaped according to a plane substantially perpendicular to its longitudinal axis, and in which these stopper-piston-valves have a central zone provided with a slit (31, 33), which is strongly compressed and closed tightly when the device is in a storage position, and which is open when one end of the transfer shaft (15) is introduced into it when the device is in said second position.

2. Device according to claim 1, characterized in that it comprises a sleeve (60) in which is located the front container (13) and at least partially the rear container (11).

3. Device according to claim 2, characterized in that the sleeve (60) is linked to a cup (70) and to a mechanism (71) for the application of multiple dosages.

4. Device according to claim 2, characterized in that said sleeve (60) is made integrally to the front container (13).

5. Device according to claim 4, characterized in that said sleeve (60) is provided with a ferrule (54) consisting of an ophthalmic drops applicator or a nasal vaporizer, integrated in the front container (13).

6. Device according to claim 2, characterized in that said sleeve includes a first tubular element (40) and a second tubular element (42) to be fitted into one another, the first tubular element containing said front container (13) and said second element containing said rear container (11).

7. Device according to claim 6, characterized in that said first tubular element has catches (43) and in that said second tubular element has at least two tongue-shaped strips (44) arranged to co-operate with said catches.

8. Device according to claim 1, characterized in that said stopper-piston-valves (19, 24) are adapted to ensure a tight and sterile closure of the corresponding containers (11, 13), by supporting the inner sides of these containers, in such a way that these containers may be stored and handled independently.

## Patentansprüche

1. Vorrichtung für die Vorbereitung einer Lösung, einer Suspension oder einer Emulsion aus einer aktiven medizinischen Substanz, die aus zwei Bestandteilen gebildet wird, wovon der eine Bestandteil als Pulver, als Lyophilisat, als Flüssigkeit oder als Paste vorliegt und der andere Bestandteil ein Verdünnungsmittel oder ein flüssiges Lösungsmittel ist, wobei die beiden Bestandteile in zwei Längsbehältern bevorratet sind, und zwar in einem distalen Behälter (11) mit verschlossenem Boden (17) und in einem proximalen Behälter (13), die jeweils an einem ihrer Enden eine Öffnung aufweisen, die durch einen Kolbenschieber, der in dem zugehörigen Behälter axial beweglich ist, verschlossen ist, wobei diese beiden Kolbenschieberverschlüsse (19,24) durch eine Übertragungsspindel (15) miteinander verbunden sind, die eingerichtet ist, um eine starre, dichte und sterile Koppelungsverbindung zwischen den beiden Kolbenschieberverschlüssen (19,24) sicherzustellen, wenn die Vorrichtung (10) sich in einer ersten, sogenannten Vorratsposition befindet und um einen dichten und sterilen Verbindungskanal (16) zwischen den beiden Behältern (11,13) zu bilden, wenn die Vorrichtung (10) sich in einer zweiten, sogenannten Einsatzposition befindet, wobei die beiden Kolbenschieberverschlüsse (19,24) jeweils eine zentrale Aussparung (25,26) aufweisen, in die ein Ansatz (27,28), der mit der Übertragungsspindel (15) fest verbunden ist, eingeführt ist, wobei die Übertragungsspindel (15) mit einem ersten Anschlag (34) und mit einem zweiten Anschlag (35) versehen ist, die außer in der Vorratsposition jeweils gegen die Seitenflächen gegenüber den Kolbenschieberverschlüssen (24) und (19) aufliegen, und in der der distale Behälter (11) den Bestandteil als Pulver, als Lyophilisat, als Flüssigkeit oder als Paste enthält und der proximale Behälter (13) den Bestandteil als Verdünnungsmittel oder als flüssiges Lösungsmittel (14) enthält, in der der distale Behälter einen größeren Querschnitt als der proximale Behälter hat, wobei der Kolbenschieberverschluß (19) des distalen Behälters (11) einen größeren Querschnitt als der Kolbenschieberverschluß (24) des proximalen Behälters (13) hat, in der die Enden der Übertragungsspindel (15) in einer im wesentlichen senkrechten Ebene zu ihrer Längsachse geschnitten sind, und in der die Kolbenschieberverschlüsse einen mittleren Bereich aufweisen, der mit einem Spalt (31,33) versehen ist, der stark zusammengedrückt und dicht verschlossen ist, wenn die Vorrichtung sich in der Vorratsposition befindet, und offen ist, wenn ein Ende der Übertragungsspindel (15) hineingeführt wird, wobei die Vorrichtung sich in der zweiten Position befindet.

2. Vorrichtung nach Anspruch 1,
**dadurch gekennzeichnet,** daß
sie eine Muffe (60) enthält, in der der proximale Behälter (13) und wenigstens teilweise der distale Behälter (11) eingesetzt sind.

3. Vorrichtung nach Anspruch 2,
**dadurch gekennzeichnet,** daß
die Muffe (60) mit einer Stoßkappe (70) und mit einer Dosiereinrichtung (71) für mehrfache Dosen verbunden ist.

4. Vorrichtung nach Anspruch 2,
**dadurch gekennzeichnet,** daß
die Muffe (60) durch das Formen zusammen mit dem proximalen Behälter (13) einteilig ausgeführt ist.

5. Vorrichtung nach Anspruch 4,
**dadurch gekennzeichnet,** daß
die Muffe (60) mit einem Ansatz (54) versehen ist, der einen Verteiler von Augentropfen oder einen Nasenzerstäuber bildet, der in dem proximalen Behälter (13) integriert ist.

6. Vorrichtung nach Anspruch 2,
**dadurch gekennzeichnet,** daß
die Muffe aus einem ersten röhrenförmigen Teil (40) und aus einem zweiten röhrenförmigen Teil (42) besteht, die ineinander gesteckt werden können, wobei der erste röhrenförmige Teil den proximalen Behälter (13) enthält und der zweite röhrenförmige Teil den distalen Behälter (11) enthält.

7. Vorrichtung nach Anspruch 6,
**dadurch gekennzeichnet,** daß
der erste röhrenförmige Teil Kerben (43) aufweist, und daß der zweite röhrenförmige Teil mindestens zwei Zungen (44) besitzt, die eingerichtet sind, um mit den Kerben zusammenzuwirken.

8. Vorrichtung nach Anspruch 1,
**dadurch gekennzeichnet,** daß
die Kolbenschieberverschlüsse (19,24) eingerichtet sind, um durch ein Anliegen gegen die Innenwandungen der Behälter (11,13) deren dichtes und steriles Schließen derart sicherzustellen, daß diese Behälter getrennt einlagerbar und verarbeitbar sind.
